# EUROPÄISCHE PATENTSCHRIFT

(11) **EP 1 061 976 B1**
(45) Veröffentlichungstag und Bekanntmachung des Hinweises auf die Patenterteilung: **08.10.2003**
(21) Anmeldenummer: 99963270.6
(22) Anmeldetag: 27.11.1999
(51) Int. Cl.: A61M 5/00, A61M 5/142, A61M 5/168, F04B 43/12, A61M 3/02

(54) **INJEKTOR ZUR APPLIZIERUNG VON FLÜSSIGKEITEN MIT EINEM DRUCKMESSSYSTEM**
INJECTOR FOR APPLYING FLUIDS FITTED WITH A PRESSURE MEASURING SYSTEM
INJECTEUR POUR L'APPLICATION DE LIQUIDES AU MOYEN D'UN SYSTEME MANOMETRIQUE

(30) Priorität: 13.01.1999 DE 19900937
(43) Veröffentlichungstag der Anmeldung: 27.12.2000
(73) Patentinhaber: Ulrich GmbH & Co. KG, 89081 Ulm (DE)
(72) Erfinder: FUTTERKNECHT, Hans-Dieter, D-89075 Ulm (DE)
(74) Vertreter: Hentrich, Swen Dipl.-Phys. Dr.
(86) Internationale Anmeldenummer: DE9903794
(87) Internationale Veröffentlichungsnummer: WO00041747

(56) Entgegenhaltungen:
- EP-A- 0 371 507
- EP-A- 0 374 858
- EP-A- 0 375 673
- DE-C- 19 525 926
- US-A- 3 807 389
- US-A- 4 398 542

## Beschreibung

Die Erfindung betrifft einen Injektor zur Applizierung von Flüssigkeiten, insbesondere von Kontrastmitteln für die Röntgen- oder Kernspintomographie, mit einem Schlauch sowie einer in Umfangsrichtung zumindest teilweise von dem Schlauch umschlungenen Rollenpumpe zur Förderung der Flüssigkeit von einem Vorratsgefäß zu einer Kanüle.

Bei derartigen aus der Praxis bekannten Injektoren werden Rollenpumpen benutzt, um die Flüssigkeit, die sich im Inneren des Schlauches befindet, ohne unmittelbaren Kontakt und daher unter Gewährleistung der Sterilität von dem Vorratsgefäß zu einem Patienten zu fördern. Dabei hat es sich als problematisch gezeigt, daß die Rollenpumpe nicht schlupffrei arbeitet, und daß die Förderleistung von dem Gegendruck abhängig ist, so daß insbesondere bei Verwendung ungeeinigter Kanülen mit zu geringem Durchmesser nicht das für die Untersuchung notwendige Flüssigkeitsquantum dem Patienten injiziert wird, obwohl die Rollenpumpe mit der gewählten, vorbestimmten Antriebsleistung bzw. Drehzahl arbeitet.

Der Erfindung liegt daher die Aufgabe zugrunde, einen Injektor der eingangs genannten Art so auszubilden, daß während der Applizierung von Flüssigkeiten jederzeit sichergestellt ist, daß der Patient die für die Untersuchung notwendige Flüssigkeitsmenge erhält.

Diese Aufgabe wird bei einem Injektor der eingangs genannten Art dadurch gelöst, daß eine durch eine in der Schlauchwandung ausgebildete Öffnung mit dem Inneren des Schlauches verbundene Druckkammer vorhanden ist, die ein unter der Wirkung des Flüssigkeitsdruckes verstellbares Bauteil aufweist, das auf einen Drucksensor einwirkt.

Diese Erfindung bietet den Vorteil, daß nicht unmittelbar der Volumenstrom der Flüssigkeit gemessen werden muß, weil erkannt worden ist, daß unterhalb eines Druckgrenzwertes ein ausreichend linearer Zusammenhang zwischen der Förderleistung der Rollenpumpe und deren Drehzahl gegeben ist, so daß es ausreichend ist, die Druckverhältnisse zu überwachen, um zu gewährleisten, daß der Druckgrenzwert nicht überschritten wird. Zu beachten ist auch, daß während der Druckmessung die Sterilität der Flüssigkeit weiterhin gewährleistet ist, weil kein unmittelbarer Kontakt der Flüssigkeit mit dem Drucksensor erfolgt, dem die Druckverhältnisse durch das verstellbare Bauteil vermittelt werden.

Bei einer Rollenpumpe liegt eine Rolle dem elastisch deformierbaren Schlauch an und verformt diesen, um so die stromab liegende Flüssigkeitssäule in Drehrichtung der Rollenpumpe weiter stromab zu transportieren. Die Elastizität des Schlauches beeinträchtigt aber die Genauigkeit der Druckmessung, so daß nach einer Weiterbildung der Erfindung vorgesehen ist, daß zwischen zwei Abschnitten des Schlauches ein starres, die Öffnung aufweisendes Rohr eingesetzt ist, an dem die Druckkammer befestigt ist.

Eine hinsichtlich der Einfachheit ihres Aufbaus sich auszeichnende Ausführungsform ist dadurch gekennzeichnet, daß das verstellbare Bauteil durch eine flüssigkeitsdichte, elastische Membran gebildet ist. Diese elastische, dem Drucksensor anliegende Membran wird bei steigendem Druck gedehnt bzw. bei sinkendem Druck wieder zusammengezogen, was sich dem Drucksensor mitteilt, der aufgrund der Lageänderung der Membran die Druckverhältnisse erfassen kann.

Wird über ein größeres Meßintervall eine ausreichende Meßgenauigkeit gewünscht, so ist es vorteilhaft, wenn das verstellbare Bauteil als Kolben ausgebildet ist, der mit einem gegenüber der Bodenplatte der Druckkammer abgedichteten Stempel auf den Drucksensor einwirkt. Bei dieser Ausführungsform gibt es keine prinzipbedingte Beschränkung des Verstellbereiches des Stempels bzw. des Kolbens, so daß dadurch Messungen in einem größeren Druckintervall durchgeführt werden können.

Derselbe Vorteil existiert auch, wenn das verstellbare Bauteil als gegenüber der Druckkammerwandung abdichtender Kolben ausgebildet ist, der unmittelbar auf den Drucksensor einwirkt, wobei bei dieser Ausführungsform die Einfachheit des Aufbaus noch weiter gesteigert ist.

Da der Injektor zum Einsatz im medizinischen Bereich vorgesehen ist, sind hohe Anforderungen an dessen Zuverlässigkeit zu stellen. Da die Erfassung der Druckverhältnisse mit dem Druckmeßsystem als eine Sicherheitseinrichtung aufzufassen ist, ist es vorteilhaft, wenn die Druckkammer und der zugeordnete Drucksensor zweifach vorgesehen sind, die Sicherheitseinrichtungen also redundant ausgelegt sind.

Prinzipiell ist es ausreichend, wenn die Druckkammer über eine Öffnung in der Schlauchwandung mit dem Inneren des Schlauches verbunden ist, da nach dem Prinzip der kommunizierenden Röhren sich Druckänderungen im Inneren des Schlauches auch der Druckkammer mitteilen, in dem beispielsweise bei steigenden Druck Flüssigkeit in die Druckkammer einströmt oder bei fallendem Druck die Strömungsrichtung sich umkehrt. Steht allerdings nur eine Öffnung zur Verfügung, kann es relativ lange dauern, bis sich nach einer Änderung des Druckes die Verhältnisse wieder stabilisiert haben, so daß die Zeitauflösung relativ gering ist. Um dies zu verbessern, ist vorgesehen, daß mehrere Öffnungen der Schlauchwandung der Druckkammer zugeordnet sind. Bei mehreren Öffnungen strömt die Flüssigkeit nicht nur in die Druckkammer ein und verbleibt dort stationär bei konstanten Druckverhältnissen, vielmehr findet eine Durchströmung der Druckkammer statt, bei der durch eine Öffnung die Flüssigkeit in die Druckkammer eintreten und durch eine andere Öffnung austreten kann, so daß das Druckmeßsystem Änderungen des Druckes schneller verfolgen kann. Günstig ist dabei auch, daß die Flüssigkeit in der Druckkammer kontinuierlich ausgetauscht wird, so daß bei einem Wechsel der Flüssigkeit, beispielsweise von einem Kontrastmittel zu einem anderen oder zu einer Spüllösung, nicht ein Rest der bisherigen Flüssigkeit in dem Schlauch verbleibt.

Es hat sich als günstig erwiesen, wenn die Druckkammer am kanülenseitigen Ende des Schlauches angeordnet ist, da mit der Druckmessung auch eine Detektion der Dichtigkeit des stromauf liegenden Schlauches erfolgen kann, zweckmäßigerweise also die Druckkammer möglichst weit stromab angeordnet ist.

Um die Funktionsfähigkeit und Unversehrtheit des Druckmeßsystems überprüfen zu können, ist es günstig, wenn der Schlauch mit der Druckkammer und dem Drucksensor durch einen Behälter geführt ist, in dem ein Feuchtefühler als Leckdetektor angeordnet ist.

Nach einer besonders bevorzugten Ausführungsform ist weiterhin vorgesehen, daß der Drucksensor durch einen elektrischen Druckaufnehmer gebildet ist, da so durch den Drucksensor nicht nur der momentane Druckwert angezeigt werden kann, sondern weiterhin die Möglichkeit besteht, das Signal des elektrischen Druckaufnehmers weiter auszuwerten, wozu zweckmäßigerweise eine die Pumpendrehzahl steuernde Auswerteeinheit für die vom elektrischen Druckaufnehmer erzeugten elektrischen Signale vorgesehen ist.

Im folgenden soll die Erfindung an einem in der Zeichnung dargestellten Ausführungsbeispiel näher erläutert werden; es zeigen:
- Fig. 1: die Vorderansicht einer schematischen Darstellung des erfindungsgemäßen Injektors,

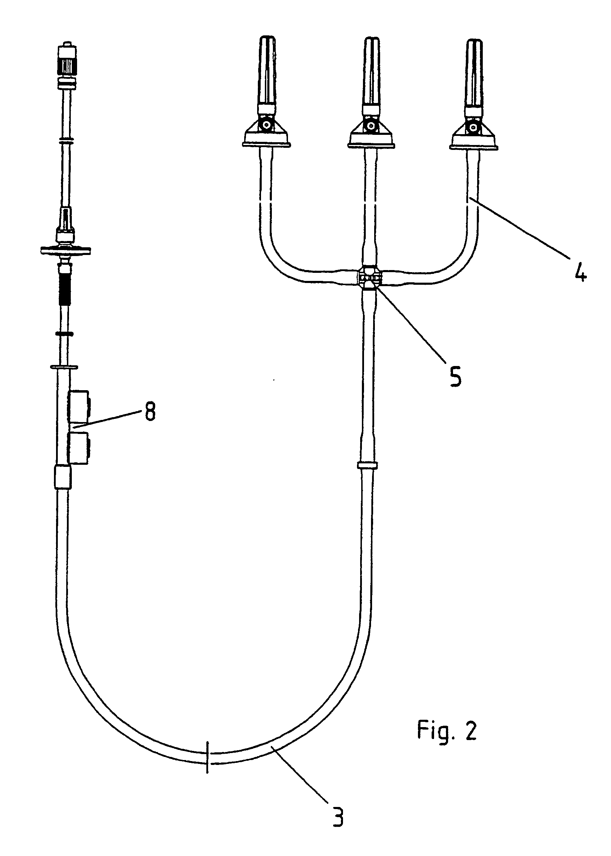

- Fig. 2: das in dem Injektor genutzte Schlauchsystem mit dem stromab angeordneten Druckmeßsystem,
- Fig. 3: das Detail III aus Fig. 1, nämlich das Druckmeßsystem mit drucklosen Druckkammern, und
- Fig. 4: eine der Fig. 3 entsprechende Darstellung mit mit dem Maximaldruck gefüllten Druckkammern.

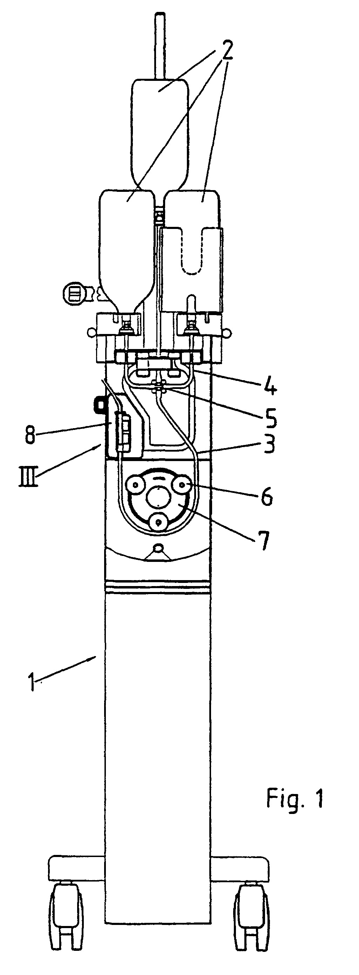

In Fig. 1 ist die Vorderansicht eines Injektors 1 gezeigt, der eine Halterung für mehrere Vorratsgefäße 2 aufweist, die über durch Absperrventile abklemmbare Verbindungsschläuche 4 und einem Verzweigungsstück 5 mit einem Schlauch 3 verbunden sind, der in Umfangsrichtung teilweise eine drei Rollen 6 aufweisende Rollenpumpe 7 umschlingt. Der Injektor 1 dient dazu, eine Flüssigkeit, insbesondere Kontrastmittel für röntgen- oder kernspintomographische Untersuchungen von einem der Vorratsgefäße 2 mittels der Rollenpumpe 7 durch den Schlauch 3 zu einem Patienten zu fördern. Um dabei sicherzustellen, daß dem Patienten stets die notwendige Menge von der Flüssigkeit injiziert wird, ist dem Injektor 1 ein Druckmeßsystem 8 zugeordnet, durch das der Druck im Inneren des Schlauches 3 erfaßbar ist. Dieses Druckmeßsystem 8 ist gebildet durch mindestens eine in der Schlauchwandung ausgebildete Öffnung 9, durch die das Innere des Schlauches 3 mit einer Druckkammer 10 verbunden ist, die ein unter der Wirkung des Flüssigkeitsdruckes verstellbares Bauteil 11 aufweist, das auf einen als elektrischen Druckaufnehmer ausgebildeten Drucksensor 12 einwirkt.

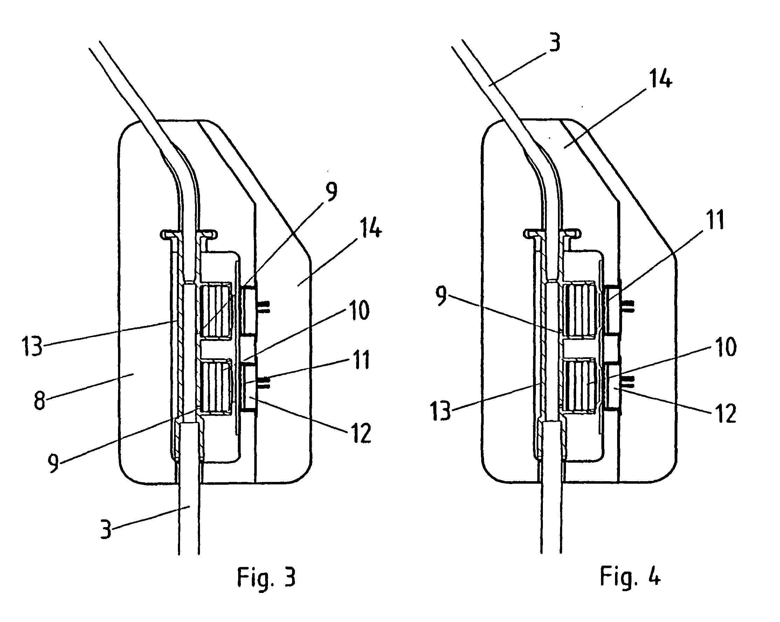

Dieses Druckmeßsystem 8 ist in den Figuren 3 und 4 als vergrößertes Detail aus der Fig. 1 dargestellt. Zwischen zwei Abschnitten des Schlauches 3 an dessen stromab gelegenen Ende ist ein starres Rohr 13 eingesetzt, das die Öffnung 9 aufweist, die in die Druckkammer 10 führen, die am Rohr 13 befestigt ist. Zu beachten ist dabei, wie aus Fig. 3 und 4 ersichtlich, daß das Druckmeßsystem 8 mit der Druckkammer 10 und dem Drucksensor 12 sowie dem verstellbaren Bauteil 11 zweifach vorgesehen ist.

Wird durch die Rollenpumpe 7 aus einem Vorratsgefäß 2 die Flüssigkeit durch den Schlauch 3 zum und durch das starre Rohr 13 gefördert, tritt die Flüssigkeit durch die Öffnung 9 in das Innere der Druckkammer 10 ein, wenn der Druck im Schlauchinneren höher als in der Druckkammer 10 ist. Steigt der Druck weiter an, wird das verstellbare Bauteil 11, das entweder als flüssigkeitsdichte, elastische Membran oder als Kolben ausgebildet sein kann, der mit einem gegenüber der Bodenplatte der Druckkammer 10 abgedichteten Stempel auf den Drucksensor 12 einwirkt, verstellt, wodurch auf den Drucksensor 12 ein größerer Druck einwirkt. Sinkt im Inneren des Schlauches 3 bzw. des starren Rohres 13 der Druck ab, strömt die Flüssigkeit aus dem Inneren der Druckkammer 10 durch die Öffnung 9 zurück, was sich durch einen am Drucksensor 12 abfallenden Druck bemerkbar macht. Sind der Druckkammer 10 zwei Öffnungen 9 zugeordnet, so wird die Druckkammer 10 kontinuierlich von der Flüssigkeit durchströmt, die nicht nur bei einem Wechsel der Druckverhältnisse in der Druckkammer 10 ausgetauscht wird.

Um eine Begrenzung des Verstellweges des verstellbaren Bauteiles 11, wie er bei der elastischen Membran systembedingt vorhanden ist, zu vermeiden, wird auch die Möglichkeit genutzt, das verstellbare Bauteil 11 als gegenüber der Druckkammerwandung abdichtender Kolben auszubilden, der unmittelbar auf den Drucksensor 12 einwirkt, was gegenüber der Ausführungsform mit Kolben und abgedichtetem Stempel den Vorteil des einfacheren Aufbaues hat.

Der Schlauch 3 und das starre Rohr 13 sind durch einen Behälter 14 geführt, in dem das Druckmeßsystem 8 mit Druckkammer 10 und dem Drucksensor 12 angeordnet ist, wobei sich weiterhin in dem Behälter 14 ein Feuchtefühler befindet, durch den austretende Flüssigkeit, die auf Undichtigkeiten des Druckmeßsystems 8 hinweist, angeordnet ist. Steigt bei einem Betrieb des Injektors 1, beispielsweise durch eine Kanüle mit zu geringem Querschnitt, der Gegendruck zu stark an, bzw. erhöht sich der Druck in dem Schlauchsystem zu stark, so daß die lineare Korrelation zwischen Drehzahl der Rollenpumpe 7 und Fördermenge nicht mehr gegeben ist, wird der gestiegene Druck durch das Druckmeßsystem 8 mit dem elektrischen Druckaufnehmer erfaßt und das Signal einer Auswerteeinheit zugeführt, die die Pumpendrehzahl steuert, wobei bei einer Überschreitung des zulässigen Druckgrenzwertes die Pumpendrehzahl soweit reduziert wird, daß der Grenzdruckwert wieder unterschritten wird und aus der Pumpendrehzahl die dem Patienten zugeführte Flüssigkeitsmenge wieder bestimmt werden kann. Die Auswerteeinheit erzeugt auch ein Warnsignal, das auf die Absenkung der Pumpendrehzahl hinweist, so daß entschieden werden kann, ob die notwendige Verlängerung der Injektionsdauer zum Erreichen des gewünschten Volumens akzeptiert werden kann, oder ein Abbruch der Untersuchung notwendig ist.

## Patentansprüche

1. Injektor zur Applizierung von Flüssigkeiten, insbesondere von Kontrastmitteln für die Röntgen- oder Kernspintomographie, mit einem Schlauch (3) sowie einer in Umfangsrichtung zumindest teilweise von dem Schlauch (3) umschlungenen Rollenpumpe (7) zur Förderung der Flüssigkeit von einem Vorratsgefäß (2) zu einer Kanüle, **dadurch gekennzeichnet, daß** eine durch eine in der Schlauchwandung ausgebildete Öffnung (9) mit dem Inneren des Schlauches (3) verbundene Druckkammer (10) vorhanden ist, die ein unter der Wirkung des Flüssigkeitsdruckes verstellbares Bauteil (11) aufweist, das auf einen Drucksensor (12) einwirkt.

2. Injektor nach Anspruch 1, **dadurch gekennzeichnet, daß** zwischen zwei Abschnitten des Schlauches (3) ein starres, die Öffnung (9) aufweisendes Rohr (13) eingesetzt ist, an dem die Druckkammer (10) befestigt ist.

3. Injektor nach Anspruch 1 oder 2, **dadurch gekennzeichnet, daß** das verstellbare Bauteil (11) durc eine flüssigkeitsdichte, elastische Membran gebildet ist.

4. Injektor nach Anspruch 1 oder 2, **dadurch gekennzeichnet, daß** das verstellbare Bauteil (11) als Kolben ausgebildet ist, der mit einem gegenüber der Bodenplatte der Druckkammer (10) abgedichteten Stempel auf den Drucksensor (12) einwirkt.

5. Injektor nach Anspruch 1 oder 2, **dadurch gekennzeichnet, daß** das verstellbare Bauteil (11) als gegenüber der Druckkammerwandung abdichtender Kolben ausgebildet ist, der unmittelbar auf den Drucksensor (12) einwirkt.

6. Injektor nach einem der Ansprüche 1 bis 5, **dadurch gekennzeichnet, daß** die Druckkammer (10) und der zugeordnete Drucksensor (12) zweifach vorgesehen sind.

7. Injektor nach einem der Ansprüche 1 bis 6, **dadurch gekennzeichnet, daß** mehrere Öffnungen (9) in der Schlauchwandung der Druckkammer (10) zugeordnet sind.

8. Injektor nach einem der Ansprüche 1 bis 7, **dadurch gekennzeichnet, daß** die Druckkammer (10) am kanülenseitigen Ende des Schlauches (3) angeordnet ist.

9. Injektor nach einem der Ansprüche 1 bis 7, **dadurch gekennzeichnet, daß** der Schlauch (3) mit der Druckkammer (10) und dem Drucksensor (12) durch einen Behälter (14) geführt ist,
in dem ein Feuchtefühler als Leckdetektor angeordnet ist.

10. Injektor nach einem der Ansprüche 1 bis 9, **dadurch gekennzeichnet, daß** der Drucksensor (12) durch einen elektrischen Druckaufnehmer gebildet ist.

11. Injektor nach Anspruch 10, **dadurch gekennzeichnet, daß** eine die Pumpendrehzahl steuernde Auswerteeinheit für die vom elektrischen Druckaufnehmer erzeugten elektrischen Signale vorgesehen ist.

## Claims

1. Injector for applying fluids, more particularly contrast agents for X-ray or nuclear magnetic tomography, comprising a hose (3) and a roller pump (7), which is at least partially enveloped by the hose (3) in the circumferential direction, for conveying the fluid from a reservoir (2) to a cannula, **characterised by** the presence of a pressure chamber (10) connected to the interior of the hose (3) by an opening (9) incorporated in the hose wall, which pressure chamber (10) features a part (11) which can be adjusted under the action of the fluid pressure and which operates on a pressure sensor (12).

2. Injector according to claim 1, **characterised in that** a rigid tube (13) incorporating the opening (9) is inserted between two sections of the hose (3) and the pressure chamber (10) is secured to said tube 13).

3. Injector according to claim 1 or 2, **characterised in that** the adjustable part (11) is formed by a fluid-tight, flexible membrane.

4. Injector according to claim 1 or 2, **characterised in that** the adjustable part (11) is configured as a piston which operates on the pressure sensor (12) by means of a plunger sealed off in relation to the baseplate of the pressure chamber (10).

5. Injector according to claim 1 or 2, **characterised in that** the adjustable part (11 ) is configured as a piston which is sealed off in relation to the walls of the pressure chamber and which operates directly on the pressure sensor (12).

6. Injector according to any of claims 1 to 5, **characterised in that** the pressure chamber (10) and the operatively associated pressure sensor (12) are provided in duplicate.

7. Injector according to any of claims 1 to 6, **characterised in that** a plurality of openings (9) in the wall of the hose are operatively associated with the pressure chamber (10).

8. Injector according to any of claims 1 to 7, **characterised in that** the pressure chamber (10) is disposed on the end of the hose (3) that is nearest to the cannula.

9. Injector according to any of claims 1 to 7, **characterised in that** the hose (3), with the pressure chamber (10) and the pressure sensor (12), passes through a container (14) in which a moisture sensor is arranged so as to detect leaks.

10. Injector according to any of claims 1 to 9, **characterised in that** the pressure sensor (12) is constituted by an electrical pressure pick-up.

11. Injector according to claim 10, **characterised in that** an evaluator which controls the pump speed is provided for the electrical signals generated by the electrical pressure pick-up.

## Revendications

1. Injecteur pour administrer des liquides, en particulier des produits de contraste pour la radiographie ou la tomographie par résonance magnétique nucléaire, comportant une tuyau souple (3) ainsi qu'une pompe à galets (7), qui est entourée au moins partiellement dans la direction périphérique par le tuyau souple (3), pour pomper le liquide d'un récipient de stockage (2) vers une canule, **caractérisé en ce qu'**il est prévu une chambre de refoulement (10) qui communique avec l'intérieur du tuyau souple (3) par l'intermédiaire d'une ouverture (9) aménagée dans la paroi du tuyau et qui présente un élément (11) déplaçable sollicité par la pression de liquide qui agit sur un capteur de pression (12).

2. Injecteur selon la revendication 1, **caractérisé en ce qu'**entre deux tronçons du tuyau souple (3) est disposé un tube (13) rigide pourvu de l'ouverture (9) auquel la chambre de pression (10) est fixée.

3. Injecteur selon la revendication 1 ou 2, **caractérisé en ce que** l'élément (11) déplaçable est formé d'une membrane élastique, étanche aux liquides.

4. Injecteur selon la revendication 1 ou 2, **caractérisé en ce que** l'élément (11) déplaçable est conformé en piston qui, avec un piston monté étanche vis-à-vis de la plaque de fond de la chambre de pression (10), agit sur le capteur de pression (12).

5. Injecteur selon la revendication 1 ou 2, **caractérisé en ce que** l'élément (11) déplaçable est conformé en piston monté étanche vis-à-vis de la paroi de la chambre de pression (10), qui agit directement sur le capteur de pression (12).

6. Injecteur selon une des revendications 1 à 5, **caractérisé en ce que** la chambre de pression (10) et le capteur de pression (12) associé sont prévus en double.

7. Injecteur selon une des revendications 1 à 6, **caractérisé en ce que** plusieurs ouvertures (9) dans la paroi du tuyau souple sont associées à la chambre de pression (10).

8. Injecteur selon une des revendications 1 à 7, **caractérisé en ce que** la chambre de pression (10) est disposée à l'extrémité côté canule du tuyau souple (3).

9. Injecteur selon une des revendications 1 à 7, **caractérisé en ce que** le tuyau souple (3) avec la chambre de pression (10) et le capteur de pression (12) sont disposés dans un récipient (14), dans lequel est placé un détecteur d'humidité en tant que détecteur de fuites.

10. Injecteur selon une des revendications 1 à 9, **caractérisé en ce que** le capteur de pression (12) est un capteur de pression électrique.

11. Injecteur selon la revendication 10, **caractérisé en ce qu'**il est prévu une unité de traitement qui contrôle la vitesse de rotation de la pompe pour les signaux électriques produits par le capteur de pression électrique.
